# EUROPEAN PATENT APPLICATION

(11) **EP 4 599 759 A1**
(43) Date of publication of application: **13.08.2025**
(21) Application number: 25154101.7
(22) Date of filing: 27.01.2025
(51) Int. Cl.: A61B 5/0205, A61B 5/024, A61B 5/08, A61B 5/00

(54) **VITAL SIGNS ESTIMATION**

(30) Priority: 07.02.2024 GB 202401623
(71) Applicant: Nokia Technologies Oy, 02610 Espoo (FI)
(72) Inventor: FARRAHI, Katayoun, Cambridge (GB); MALEKZADEH, Mohammad, Cambridge (GB)
(74) Representative: Nokia EPO representatives

(57) **Abstract**

An apparatus, method and computer program is described comprising: obtaining data (e.g. physiological data) relating to a user; estimating user profiles based on the obtained data; determining a confidence level in said estimated user profiles based on differences between said user profiles and corresponding stored data profiles for an identified activity of the user; and performing signal enhancement in the event that one or more of the estimated user profiles has a confidence level below a respective threshold level, wherein said signal enhancement generates an enhanced user profile of one of the estimated user profiles having a lower confidence level from one of the estimated user profiles having a higher confidence level and a stored cardiorespiratory coupling ratio for the user.

## Description

### Field

Example embodiments may relate to systems, methods and/or computer programs for estimating user vital signs, such as respiratory rate, heart rate and blood pressure.

### Background

Systems exist for estimating user vital sings such as respiratory rate, heart rate and blood pressure. There remains a need for further developments in this field.

### Summary

According to a first aspect, there is describes an apparatus comprising: means for obtaining data (e.g. photoplethysmography data) relating to a user, wherein said data includes physiological data; means for estimating user profiles based on the obtained data, wherein the user profiles include a user respiratory rate profile and at least one of a user heart rate profile or a user blood pressure profile; means for determining a confidence level in said estimated user profiles based on differences between said user profiles and corresponding stored data profiles for an identified activity of the user; and means for performing signal enhancement in the event that one or more of the estimated user profiles has a confidence level below a respective threshold level, wherein said signal enhancement is configured to generate an enhanced user profile of one of the estimated user profiles having a lower confidence level based, at least in part, on one of the estimated user profiles having a higher confidence level and a cardiorespiratory coupling ratio for the user. The cardiorespiratory coupling ratio may, for example, be stored as part of the respective stored user profile.

The said cardiorespiratory coupling ratio may be activity-specific and/or user-specific. For example, for a particular user, a number of cardiorespiratory coupling ratios may be stored, with each ratio being for a particular identified activity.

In some example embodiments, the means for performing signal enhancement is configured to update the estimated user profile having the lowest confidence level in the event that one or more of the estimated user profiles has a confidence level below the respective threshold level.

The respective threshold may be user profile dependent.

The means for performing signal enhancement may be configured to generate one of: an enhanced user respiratory rate profile from one of said estimated user heart profile or user blood pressure profile and said stored cardiorespiratory coupling ratio for the user; or an enhanced user heart rate profile or an enhanced user blood pressure profile from said estimated user respiratory rate profile and said stored cardiorespiratory coupling ratio.

The apparatus may further comprise one or more of: means for generating a heart rate output, wherein the heart rate output is based on said estimated user heart rate profile or, where generated, said enhanced user heart rate profile; means for generating a blood pressure output, wherein the blood pressure output is based on said estimated user blood pressure profile or, where generated, said enhanced user blood pressure profile; or means for generating a respiratory rate output, wherein the respiratory rate output is based on said estimated user respiratory rate profile or, where generated, said enhanced user respiratory rate profile.

Some example embodiments further comprise means (e.g. a human activity recognition module) for identifying said activity (such as the user being stationary, running, standing, sleeping, walking etc.). The activity may be identified based, at least in part, on motion data relating to the user.

The stored data profiles may, for example, comprise user respiratory rate data profile templates for different activities and at least one of heart rate data profile templates or blood pressure data profile templates for different activities. In some example embodiments, then means for determining said confidence level is configured to generate correlations of estimated user profiles with respective stored data profiles templates, wherein said confidence levels are based said correlations.

In some example embodiments, the means for obtaining data relating to said user comprises one or more sensors. Such sensors may include, for example, earbuds, a smartwatch or the like. Multiple sensors may be provided.

The apparatus may further comprise means for updating said stored data. Thus, a training step may be provided.

According to a second aspect, there is describes a method comprising: obtaining data relating to a user, wherein said data includes physiological data; estimating user profiles based on the obtained data, wherein the user profiles include a user respiratory rate profile and at least one of a user heart rate profile or a user blood pressure profile; determining a confidence level in said estimated user profiles based on differences between said user profiles and corresponding stored data profiles for an identified activity of the user; and performing signal enhancement in the event that one or more of the estimated user profiles has a confidence level below a respective threshold level, wherein said signal enhancement generates an enhanced user profile of one of the estimated user profiles having a lower confidence level based, at least in part, on one of the estimated user profiles having a higher confidence level and a stored cardiorespiratory coupling ratio for the user.

The said cardiorespiratory coupling ratio may be activity-specific and/or user-specific.

Performing signal enhancement may comprise updating the estimated user profile having the lowest confidence level in the event that one or more of the estimated user profiles has a confidence level below the respective threshold level.

The respective threshold may be user profile dependent.

Performing signal enhancement may comprising generating one of: an enhanced user respiratory rate profile from one of said estimated user heart profile or user blood pressure profile and said stored cardiorespiratory coupling ratio for the user; or an enhanced user heart rate profile or an enhanced user blood pressure profile from said estimated user respiratory rate profile and said stored cardiorespiratory coupling ratio.

The method may further comprising generating one or more of: a heart rate output (wherein the heart rate output is based on said estimated user heart rate profile or, where generated, said enhanced user heart rate profile); a blood pressure output (wherein the blood pressure output is based on said estimated user blood pressure profile or, where generated, said enhanced user blood pressure profile); or a respiratory rate output (wherein the respiratory rate output is based on said estimated user respiratory rate profile or, where generated, said enhanced user respiratory rate profile).

A human activity recognition (HAR) module or some similar module may be provided for identifying said activity (such as the user being stationary, running, standing, sleeping, walking etc.). The activity may be identified based, at least in part, on motion data relating to the user.

The stored data profiles may comprise user respiratory rate data profile templates for different activities and at least one of heart rate data profile templates or blood pressure data profile templates for different activities. Determining said confidence level may comprise generating correlations of estimated user profiles with respective stored data profiles templates, wherein said confidence levels are based said correlations.

The method may further comprise updating said stored data. Thus, a training step may be provided.

According to a third aspect, there is provided a computer-readable instructions which, when executed by a computing apparatus, cause the computing apparatus to perform (at least) any method as described herein (including the method of the second aspect described above).

According to a fourth aspect, there is provided a computer-readable medium (such as a non-transitory computer-readable medium) comprising program instructions stored thereon for performing (at least) any method as described herein (including the method of the second aspect described above).

According to a fifth aspect, there is provided an apparatus comprising: at least one processor; and at least one memory including computer program code which, when executed by the at least one processor, causes the apparatus to perform (at least) any method as described herein (including the method of the second aspect described above).

According to a sixth aspect, there is provided a computer program comprising instructions which, when executed by an apparatus, cause the apparatus to perform at least the following: obtain data relating to a user, wherein said data includes physiological data; estimate user profiles based on the obtained data, wherein the user profiles include a user respiratory rate profile and at least one of a user heart rate profile or a user blood pressure profile; determine a confidence level in said estimated user profiles based on differences between said user profiles and corresponding stored data profiles for an identified activity of the user; and perform signal enhancement in the event that one or more of the estimated user profiles has a confidence level below a respective threshold level, wherein said signal enhancement generates an enhanced user profile of one of the estimated user profiles having a lower confidence level based, at least in part, on one of the estimated user profiles having a higher confidence level and a stored cardiorespiratory coupling ratio for the user.

According to a seventh aspect, there is provided an apparatus comprising: sensors (or some other means) for obtaining data relating to a user, wherein said data includes physiological data; a data estimation module (or some other means) for estimating user profiles based on the obtained data, wherein the user profiles include a user respiratory rate profile and at least one of a user heart rate profile or a user blood pressure profile; a cross-correlation module (or some other means) for determining a confidence level in said estimated user profiles based on differences between said user profiles and corresponding stored data profiles for an identified activity of the user; and a signal enhancement module (or some other means) for performing signal enhancement in the event that one or more of the estimated user profiles has a confidence level below a respective threshold level, wherein said signal enhancement generates an enhanced user profile of one of the estimated user profiles having a lower confidence level based, at least in part, on one of the estimated user profiles having a higher confidence level and a cardiorespiratory coupling ratio for the user.

### Brief Description of the Drawings

Example embodiments will now be described by way of non-limiting example, with reference to the accompanying drawings, in which:
FIG. 1 is a block diagram of a system in which example embodiments described herein may be used;
FIG. 2 is a block diagram of a system in accordance with an example embodiment;
FIG. 3 is a flow chart showing an algorithm in accordance with an example embodiment;
FIG. 4 is a block diagram of a system in accordance with an example embodiment;
FIG. 5 is a flow chart showing an algorithm in accordance with an example embodiment;
FIG. 6 is a block diagram of components of a system in accordance with an example embodiment; and
FIG. 7 shows an example of tangible media for storing computer-readable code which when run by a computer may perform methods according to example embodiments described above.

### Detailed Description

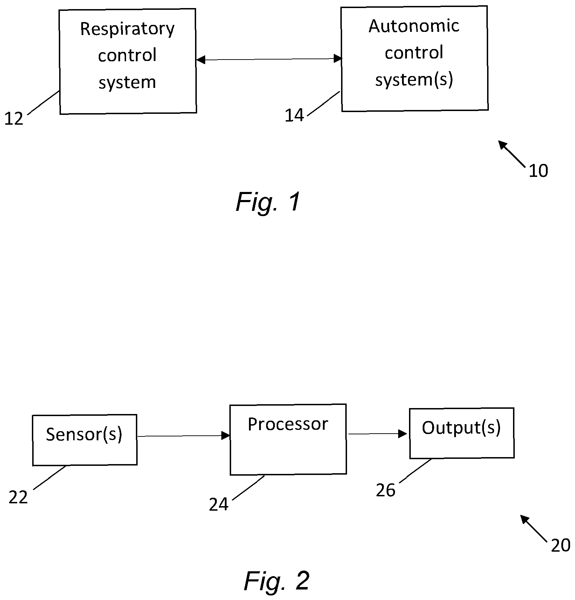

FIG. 1 is a block diagram of a system, indicated generally by the reference numeral 10, in which example embodiments described herein may be used. The system 10 comprises a respiratory control system 12 and an autonomic control system 14.

Cardiorespiratory coupling describes the influences of respiration on heart rate (HR) and blood pressure (BP). The cardiorespiratory coupling reflects a reciprocal interaction between respiratory systems and autonomic systems, such as the systems 12 and 14.

In simpler terms, cardiorespiratory coupling may refer to the influence of the arterial pulse pressure on respiration and relates to the tendency for the next inhalation in a breathing cycle to start at a preferred latency after the last heartbeat in exhalation. Thus, breathing biomarkers, including respiratory rate (RR), fractional inspiratory time (FIT) and inhalation-exhalation ratio (IER) are influenced by the pulse, and vice versa.

Aspects of the system 10 that are relevant to some example embodiments described herein include:
- Breath rhythm has an impact on heart rate and blood pressure. For example, the rate at which inhalation and exhalation occurs has an impact on how fast the heart beats and how hard our blood is pumped around the arteries of the body.
- There is a mutual or reciprocal interaction between the autonomic cardiovascular system 14 (which controls involuntary bodily functions like heart rate) and the respiratory control system 12 (which regulates breathing).
- Not only does breathing affect the cardiovascular system, but the cardiovascular system can also influence breathing patterns. In other words, the way our heart pumps blood can affect how we breathe.
- There may be a specific timing or delay between the last heartbeat during exhalation and the beginning of the next inhalation. In cardiorespiratory coupling, there may be a preferred or regular timing for when the next breath starts after your last heartbeat during exhalation. This is sometimes referred to as "preferred latency".
- Breathing biomarkers are characteristics of our breathing. Breathing biomarkers such as the rate at which we breathe (respiratory rate, RR), the time spent inhaling compared to the entire breathing cycle (fractional inspiratory time, FIT), and the ratio of inhalation to exhalation (inhalation-exhalation ratio, IER), are influenced by the pulsing of our arteries (the pulse). Conversely, these aspects of our breathing can also influence our pulse and heart rate.

FIG. 2 is a block diagram of a system, indicated generally by the reference numeral 20, in accordance with an example embodiment. The system 20 comprises one or more sensors 22, a processor 24 and one or more outputs 26.

The sensor(s) 22 may, for example, include one or more of smart devices such as smartwatches, smart rings, and earbuds that are used to measure vital signs data such as: pulse or heart rate; blood pressure; respiratory rate etc. Measuring vital signs in an accurate manner can help us better understand the health and wellbeing state of the user.

The processor 24 may make use of the principles of cardiorespiratory coupling to generate estimates of one or more vital signs data based on one or more other vital signs data. Vital signs data (whether measured or estimated) may be provided as the output(s) 26. These estimates may, for example, be made using photoplethysmography (PPG), which is a technique that measures reflected light that is proportional to blood volume variations, from skin.

It is not trivial to accurately estimate vital signs, especially when they are outside the normal range. For an adult, for example, the normal range may be considered to be a respiratory rate of the order of 12-20 breaths per minute and a pulse rate of the order of 60-100 beats per minute.

The complexity in predicting vital signs is compounded by the fact that these measurements are dynamic and change over time. This dynamic nature makes it challenging to predict vital signs accurately over extended periods. Moreover, the connection between vital signs can be influenced by various factors that can vary from one individual to another, or from one scenario to another.

Furthermore, there are numerous sources of potential error in vital sign estimations. These errors can be caused by factors like motion (e.g., if the person wearing the device is moving), skin tone, hair, how tightly or loosely the sensor is worn (e.g., a loose-fitting watch), or background noise (e.g., when a microphone is used for estimation). These sources of error can impact the reliability of the predictions or estimates made using smart devices as sensors.

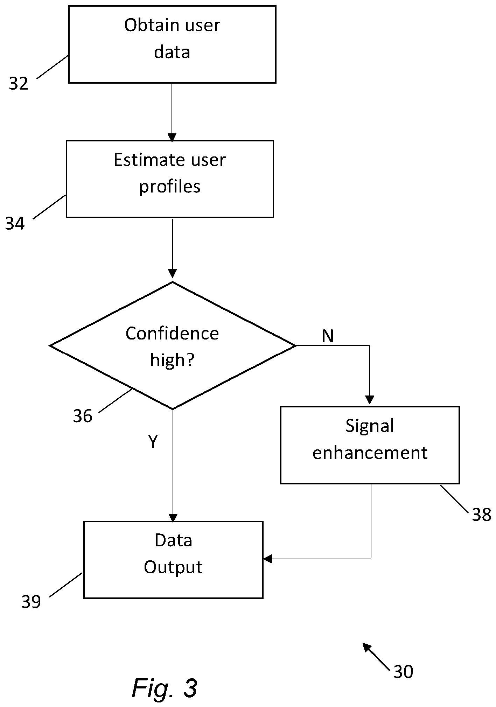

FIG. 3 is a flow chart showing an algorithm, indicated generally by the reference numeral 30, in accordance with an example embodiment. The algorithm 30 may be implemented by the system 20 described above, by the system 40 described below, or by some similar system. For example, the operation 32 may be implemented by the sensors 22, the operations 34, 36 and 38 may be implemented by the processor 24, and the operation 39 may provide the output 26 of the system 20 described above.

The algorithm 30 starts at operation 32, where physiological data (e.g. photoplethysmography data) relating to a user is obtained. The data may be obtained by the sensor(s) 22 described above.

At operation 34, user profiles are estimated based on the data obtained in the operation 32. As discussed in detail below, the estimated user profiles include profiles such as a user respiratory rate profile, a user heart rate profile and/or a user blood pressure profile. For example, the estimated user profiles may comprise a user respiratory rate profile and at least one of a user heart rate profile or a user blood pressure profile.

At operation 36, confidence levels in the user profiles estimated in the operation 34 are determined. The confidence levels may be based on differences between said user profiles and corresponding stored data profiles for an identified activity of the user. For example, as discussed in detail below, the confidence level(s) may be based on cross-correlations of estimated user profiles with respective user data profiles templates.

If the confidence levels are determined to be high, then the algorithm moves to operation 39; otherwise, the algorithm moves to operation 38.

At operation 38, signal enhancement is performed in the event that one or more of the user profiles estimated in the operation 34 is determined, in the operation 36, to have a confidence level below a threshold level (which threshold level may be user profile dependent). Thus, none of the estimated user profiles is typically updated if all user profiles have a confidence level above the relevance threshold level. It should be noted that the confidence levels are typically short-term or instant confidence levels, rather than being confidence levels over the entire duration of an activity. Thus, the algorithm 30 is intended to run on short intervals or batches of physiological data, for example by repeatedly performing signal enhancement where confidence in the accuracy of a data point is low.

The signal enhancement performed in the operation 38 may generate an enhanced vital sign estimation from one of the estimated user profiles having a lower confidence level from one of the estimated user profiles having a higher confidence level and a cardiorespiratory coupling ratio for the user (e.g. as stored as part of the respective stored data profile).

In one example embodiment, the signal enhancement of operation 38 may include generating an enhanced user respiratory rate profile from either an estimated user heart profile or an estimated user blood pressure profile and the stored cardiorespiratory coupling ratio for the user. Alternatively, or in addition, the signal enhancement of operation 38 may include generating an enhanced user heart rate profile or an enhanced user blood pressure profile from said estimated user respiratory rate profile and said stored cardiorespiratory coupling ratio. Thus, the signal enhancement may make use of the linkage between respiratory and autonomic control signal as expressed by the cardiorespiratory coupling ratio to generate an estimate of data from other, potentially more reliable, data.

At operation 39, one or more data outputs are provided. The data outputs may be the user profiles (e.g. estimated and or updated user profiles). Alternatively, or in addition, the data outputs may include vital signs data (such as one or more of heart rate, blood pressure or respiratory rate) based on said user profiles. In some example embodiments, the operation 39 is omitted (with the generated user profiles being used in some other way).

By way of example, the operation 39 may generate one or more of:
- A heart rate output based on an estimated user heart rate profile (as generated, for example, during the operation 34) or, where generated, said enhanced user heart rate profile (as generated, for example, during the operation 38);
- A blood pressure output based on said estimated user blood pressure profile (as generated, for example, during the operation 34) or, where generated, said enhanced user blood pressure profile (as generated, for example, during the operation 38); or
- A respiratory rate output based on said estimated user respiratory rate profile (as generated, for example, during the operation 34) or, where generated, said enhanced user respiratory rate profile (as generated, for example, during the operation 38).

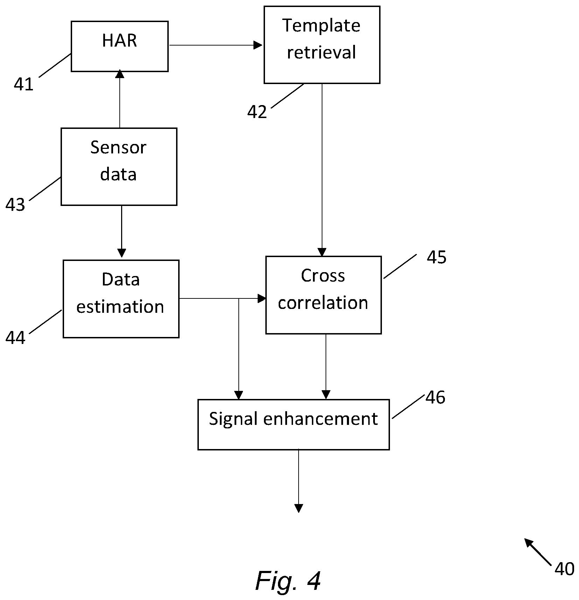

FIG. 4 is a block diagram of a system in accordance with an example embodiment. As noted above, the system 40 may be used to implement aspects of the algorithm 30 described above.

The system 40 comprising a human activity recognition (HAR) module 41, a template retrieval module 42, a sensor data input 43 (which may be used to implement the operation 32 described above), a data estimation module 44 (which may be used to implement the operation 34 described above), a cross-correlation module 45 (which may be used to implement the operation 36 described above), and a signal enhancement module 46 (which may be used to implement the operation 38 described above).

The human activity recognition (HAR) module 41 is used to identify a current activity (denoted herein by *α*) of a user (e.g. running, walking, stationary etc.) based, for example, on the sensor data 43. Determining the user activity may be useful since the cardiorespiratory coupling rate may be activity-specific (as well as being user specific).

The template retrieval module 42 obtains stored data profiles (and potentially other statistics) for some or all of respiratory rate, heart rate or blood pressure based on the activity α as detected by the HAR module 41. In one example embodiment, the template retrieval module 42 obtains a user respiratory rate data profile template and at least one of heart rate data profile templates or blood pressure data profile templates for the determined activity.

By way of example, the template retrieval module may obtain the following data for the identified activity:
- HR/RR ratio (Γ_{α});
- A signal template for respiratory rate (ω̅_{α*_{RR}*});
- A signal template for heart rate (ω̅_{α*_{HR}*}) and/or blood pressure (ω̅_{α*_{BP}*}); and
- Standard deviations of the obtained signal template (σ̅_{α*_{RR}*}, σ̅_{α*_{HR}*} and/or σ̅_{α*_{BP}*}).

The sensor data input 43 obtains physiological data (denoted herein by s), such as PPG signals. The sensor input data may be obtained from sensors such as a smartwatch, smartphone, earbuds or the like.

The data estimation module 44 estimates user profile data such as a respiratory rate data signal (ω̅*_{RR}*), a heart rate data signal (ω̅*_{HR}*) or a blood pressure signal (ω̅*_{BP}*). In one example embodiment, the data estimation module 44 estimates a respiratory rate user profile (ω̅*_{RR}*) and one or more of a heart rate user profile (ω̅*_{HR}*) or a blood pressure user profile (ω̅*_{BP}*).

The cross-correlation module 45 computes cross-correlations between user profile data provided by the data estimation module and corresponding stored data profile provided by the template retrieval module 42 (for the identified activity). The cross-correlation module 45 thereby determines the differences between the estimated and stored user profiles that can be used to determine a confidence level of the estimated profiles.

For example, the cross-correlation module may compute a cross correlation between one or more of:
- The estimated respiratory rate data signal (ω̅*_{RR}*) and the signal template for respiratory rate (ω̅_{α*_{RR}*}) for the identified activity.
- The estimated heart rate data signal (ω̅*_{HR}*) and the signal template for heart rate *(*ω̅_{α*_{HR}*}) for the identified activity.
- The estimated blood pressure data signal (ω̅*_{BP}*) and the signal template for blood pressure (ω̅_{α*_{BP}*}) for the identified activity.

The confidence levels discussed above can then be determined based on the determined correlations. Specifically, the confidence levels can be determined based on whether the respective cross-correlations are greater than the standard deviation as obtained by the template retrieval module 42.

The signal enhancement module 46 performs signal enhancement based on the computed uncertainties. For example, the signal enhancement module may update the estimated user profile (as generated by the data estimation module 44) having the lowest confidence level in the event that one or more of the estimated user profiles has a confidence level below a respective threshold level.

Thus, for example, if the differences in the cross-correlations discussed above are less than the corresponding standard deviation (σ̅_{α}*_{RR},* σ̅_{α*_{HR}*} and σ̅_{α*_{BP}*}), then the data obtained by the data estimation module 44 may be used (without signal enhancement); otherwise, the signal with highest uncertainty can be enhanced based on a signal with high certainty.

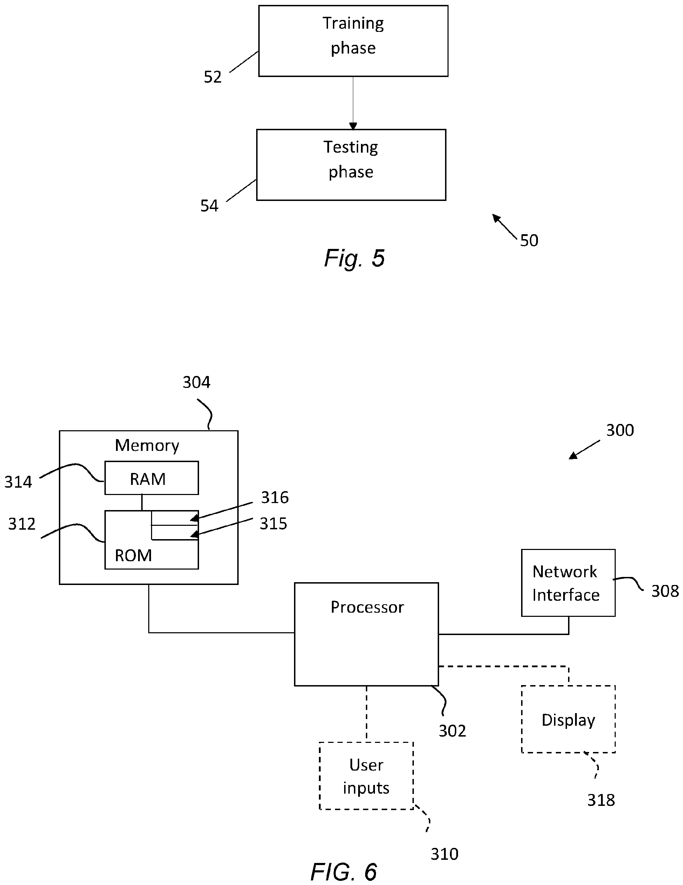

FIG. 5 is a flow chart showing an algorithm, indicated generally by the reference numeral 50, in accordance with an example embodiment. The algorithm 50 has a training phase 52 and a testing phase 54. As discussed below, the training phase enables the templates that are available to the template retrieval module 42 to be updated. The testing phase 54 may be implemented using the system 40 described above.

The training phase 52 may include the following steps:
- For a range of activities, e.g. α = {stationary, walking, running}, store the HR/RR ratio Gₐ. This ratio may be computed based on ground truth data collected from various number of users. The ratio may, for example, be an average.
- For each of these activities, store as a template, the time varying sensor signal wₐ for two phases (e.g. two breaths or two heart beats by considering the peaks and troughs of the signals) of the RR w_{a_RR} and HR w_{a_HR}.
- Consider multiple occurrences of w_{a_RR} and w_{a_HR}, and store the standard deviation of the cross correlation between them as s_{a_RR} and s_{a_HR}.

The testing phase 54 may include the following steps:
- Compute the activity for the given instant in time aₜ.
- Consider the first two phases of the vital signs from the sensor signals and compute the cross correlation with the respective templates (e.g. the template w_{a_RR} and w_{a_HR} where respiratory rate and heart rate are being considered.
- If the difference in the cross correlation is less than the standard deviation s_{a_RR} and s_{a_HR} for both signals (indicating that the confidence level is high) then estimate the vital signs (hear rate and respiratory rate in this example) using the sensor signal(s).
- If the difference in the cross correlation is greater than the standard deviation s_{a_RR} and s_{a_HR} for only one of the signals (indicating that the confidence level in that one signal is low), then enhance the estimation of the signal (e.g. heart rate or respiratory rate in this example) having the larger standard deviation using the other estimated signal and the cardiorespiratory coupling Gₐ for the activity.
- If both signals have greater standard deviations than the templates for the given activity (indicating that the confidence level is both signal is low), then use the signal that has the lower standard deviation as the more certain one and enhance the other signal's vital using the cardiorespiratory coupling Gₐ for the activity.

The training phase 52 may be implemented from time-to-time (e.g. once a month). This enables the accuracy of the stored templates to be updated.

The testing phase may be implemented, for example, every few minutes. This enables vital signs data to be kept regularly updated, if necessary.

The methodology behind the example embodiments described above is to store a user profile of HR/RR ratio over a range of activities for a user, where the activities are determined by human activity recognition using, for example, HAR technology.

The HR/RR ratio is typically individual to a user for various activities and is therefore computed and stored to use for enhancement of a signal when high uncertainty of a signal arises. A template of the sensor signal for each vital sign from the peak to the next peak of the signal may be stored (for example for each activity) as well as a standard deviation value of the cross correlation between templates of the sensor signal for the given activity. This can be used to compute the uncertainty of the sensor signal for an activity, as discussed above. If enhancement is carried out, the less uncertain vital sign sensor system may be used, in addition to the user profile information, to enhance the estimation of the more uncertain vital sign using the HR/RR ratio.

For completeness, FIG. 6 is a schematic diagram of components of one or more of the example embodiments described previously, which hereafter are referred to generically as a processing system 300. The processing system 300 may, for example, be the apparatus referred to in the claims below.

The processing system 300 may have a processor 302, a memory 304 closely coupled to the processor and comprised of a RAM 314 and a ROM 312, and, optionally, a user input 310 and a display 318. The processing system 300 may comprise one or more network/apparatus interfaces 308 for connection to a network/apparatus, e.g. a modem which may be wired or wireless. The network/apparatus interface 308 may also operate as a connection to other apparatus such as device/apparatus which is not network side apparatus. Thus, direct connection between devices/apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 304 stores, amongst other things, an operating system 315 and may store software applications 316. The RAM 314 of the memory 304 is used by the processor 302 for the temporary storage of data. The operating system 315 may contain code which, when executed by the processor implements aspects of the algorithms 30 and 50 described above. Note that in the case of small device/apparatus the memory can be most suitable for small size usage i.e. not always a hard disk drive (HDD) or a solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/apparatus such as IoT device/apparatus i.e. embedded to very small size.

In some example embodiments, the processing system 300 may also be associated with external software applications. These may be applications stored on a remote server device/apparatus and may run partly or exclusively on the remote server device/apparatus. These applications may be termed cloud-hosted applications. The processing system 300 may be in communication with the remote server device/apparatus in order to utilize the software application stored there.

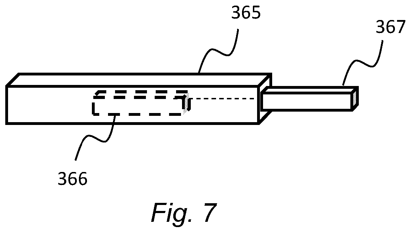

FIG. 7 shows a tangible media, in the form of a removable memory unit 365, storing computer-readable code which when run by a computer may perform methods according to example embodiments described above. The removable memory unit 365 may be a memory stick, e.g. a USB memory stick, having internal memory 366 storing the computer-readable code. The internal memory 366 may be accessed by a computer system via a connector 367. Of course, other forms of tangible storage media may be used, as will be readily apparent to those of ordinary skilled in the art. Tangible media can be any device/apparatus capable of storing data/information which data/information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices/apparatus and other devices/apparatus. References to computer program, instructions, code etc. should be understood to express software for a programmable processor firmware such as the programmable content of a hardware device/apparatus as instructions for a processor or configured or configuration settings for a fixed function device/apparatus, gate array, programmable logic device/apparatus, etc.

If desired, the different functions discussed herein may be performed in a different order and/or concurrently with each other. Furthermore, if desired, one or more of the above-described functions may be optional or may be combined. Similarly, it will also be appreciated that the flow diagrams of Figures 3 and 5 are examples only and that various operations depicted therein may be omitted, reordered and/or combined.

It will be appreciated that the above-described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the above describes various examples, these descriptions should not be viewed in a limiting sense. Rather, there are several variations and modifications which may be made without departing from the scope of the present invention as defined in the appended claims.

## Claims

1. An apparatus comprising:
means for obtaining data relating to a user, wherein said data includes physiological data;
means for estimating user profiles based on the obtained data, wherein the user profiles include a user respiratory rate profile and at least one of a user heart rate profile or a user blood pressure profile;
means for determining a confidence level in said estimated user profiles based on differences between said user profiles and corresponding stored data profiles for an identified activity of the user; and
means for performing signal enhancement in the event that one or more of the estimated user profiles has a confidence level below a respective threshold level, wherein said signal enhancement is configured to generate an enhanced user profile of one of the estimated user profiles having a lower confidence level based, at least in part, on one of the estimated user profiles having a higher confidence level and a stored cardiorespiratory coupling ratio for the user.

2. An apparatus as claimed in claim 1, wherein said cardiorespiratory coupling ratio is activity-specific.

3. An apparatus as claimed in claim 1 or claim 2, wherein the means for performing signal enhancement is configured to update the estimated user profile having the lowest confidence level in the event that one or more of the estimated user profiles has a confidence level below the respective threshold level.

4. An apparatus as claimed in any one of claims 1 to 3, wherein the respective threshold is user profile dependent.

5. An apparatus as claimed in any one of the preceding claims, wherein said means for performing signal enhancement is configured to generate one of:
an enhanced user respiratory rate profile from one of said estimated user heart profile or user blood pressure profile and said stored cardiorespiratory coupling ratio for the user; or
an enhanced user heart rate profile or an enhanced user blood pressure profile from said estimated user respiratory rate profile and said stored cardiorespiratory coupling ratio.

6. An apparatus as claimed in claim 5, further comprising:
means for generating a heart rate output, wherein the heart rate output is based on said estimated user heart rate profile or, where generated, said enhanced user heart rate profile.

7. An apparatus as claimed in claim 5 or claim 6, further comprising:
means for generating a blood pressure output, wherein the blood pressure output is based on said estimated user blood pressure profile or, where generated, said enhanced user blood pressure profile.

8. An apparatus as claimed in any one of claims 5 to 7, further comprising:
means for generating a respiratory rate output, wherein the respiratory rate output is based on said estimated user respiratory rate profile or, where generated, said enhanced user respiratory rate profile.

9. An apparatus as claimed in any one of the preceding claims, further comprising means for identifying said activity.

10. An apparatus as claimed in any one of the preceding claims, wherein said stored data profiles comprise user respiratory rate data profile templates for different activities and at least one of heart rate data profile templates or blood pressure data profile templates for different activities.

11. An apparatus as claimed in claim 10, wherein said means for determining said confidence level is configured to generate correlations of estimated user profiles with respective stored data profiles templates, wherein said confidence levels are based said correlations.

12. An apparatus as claimed in any one of the preceding claims, wherein the means for obtaining data relating to said user comprises one or more sensors.

13. An apparatus as claimed in any one of the preceding claims, further comprising:
means for updating said stored data.

14. A method comprising:
obtaining data relating to a user, wherein said data includes physiological data;
estimating user profiles based on the obtained data, wherein the user profiles include a user respiratory rate profile and at least one of a user heart rate profile or a user blood pressure profile;
determining a confidence level in said estimated user profiles based on differences between said user profiles and corresponding stored data profiles for an identified activity of the user; and
performing signal enhancement in the event that one or more of the estimated user profiles has a confidence level below a respective threshold level, wherein said signal enhancement generates an enhanced user profile of one of the estimated user profiles having a lower confidence level based, at least in part, on one of the estimated user profiles having a higher confidence level and a stored cardiorespiratory coupling ratio for the user.

15. A computer program comprising instructions which, when executed by an apparatus, cause the apparatus to perform at least the following:
obtain data relating to a user, wherein said data includes physiological data;
estimate user profiles based on the obtained data, wherein the user profiles include a user respiratory rate profile and at least one of a user heart rate profile or a user blood pressure profile;
determine a confidence level in said estimated user profiles based on differences between said user profiles and corresponding stored data profiles for an identified activity of the user; and
perform signal enhancement in the event that one or more of the estimated user profiles has a confidence level below a respective threshold level, wherein said signal enhancement generates an enhanced user profile of one of the estimated user profiles having a lower confidence level based, at least in part, on one of the estimated user profiles having a higher confidence level and a stored cardiorespiratory coupling ratio for the user.
